(19) European Patent Office / Europäisches Patentamt / Office européen des brevets

(11) **EP 1 386 962 A1**

(12) **EUROPEAN PATENT APPLICATION**

(43) Date of publication:
**04.02.2004 Bulletin 2004/06**

(51) Int Cl.$^7$: **C12N 9/12**, C12P 19/34, C12P 21/02

(21) Application number: **02291959.1**

(22) Date of filing: **02.08.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(71) Applicant: **Proteus**
**30000 Nîmes (FR)**

(72) Inventors:
• **Ryabova, Lyubov**
  **30900 Nîmes (FR)**
• **Masson, Jean-Michel**
  **31400 Toulouse (FR)**

(74) Representative: **Breesé, Pierre**
**BREESE - MAJEROWICZ,**
**3, avenue de l'Opéra**
**75001 Paris (FR)**

(54) **Methods and compositions for in vitro synthesis of biological macromolecules in a cell-free system enriched with atp-sulfurylase**

(57) The present invention provides methods and composition for enhancing *in vitro* synthesis of biological macromolecules in a cell-free system where ATP is required as a primary energy source, wherein said cell-free system is enriched with ATP-sulfurylase

The invention relates also to as cell-free systems and cell-free extracts enriched with ATP-sulfurylase for enhancing *in vitro* synthesis of any biological macromolecules.

EP 1 386 962 A1

**Description**

The present invention pertains to the field of methods and compositions that enhance *in vitro* synthesis of biological macromolecules such as nucleic acids and proteins in a cell-free system.

<u>**Background.**</u>

In recent years, *in vitro* synthesis have been considered as an alternative to the conventional recombinant DNA technology, because of relatively high level of nucleic acid and protein production free from disadvantages associated with cellular expression. *In vivo* protein expression, being a powerful tool, has a set of significant limitations : (1) proteins produced *in vivo* could be subjected to degradation or post-translational modifications during cellular expression, such as glycosylation, deamination or oxidation; (2) cytotoxic or insoluble proteins may affect their synthesis and inhibit metabolic processes and the viability of the cell. To overcome these problems *in vitro* protein synthesis is mainly applied to (1) expression of toxic proteins; (2) radiolabeling of newly synthesized proteins and incorporation of unnatural amino acids; (3) screening quickly and economically for pharmaceuticals, food, environmental and commodity products.

The synthesis of proteins and other biological macromolecules in a cell-free system is based on cell extract. A typical *in vitro* cell-free system comprises : (1) a crude cell extract which contains the enzymes and factors necessary for transcription and translation [Zubay G: *In vitro* synthesis of proteins in microbial system, *Annu Rev Genet* 1973, 7: 267-287], (2) NTPs, (3) an energy regeneration system like acetyl phosphate (AcPh) and acetate kinase, phosphoenolpyruvate (PEP) and pyruvate kinase or creatine phosphate (CrPh) and creatine kinase, and (4) DNA and RNA.

However so called batch mode of cell-free gene expression systems has not been widely accepted as a practical alternative due to the short time of protein synthesis. After the development of a continuous flow cell-free (CFCF) system by Spirin et al. (Science 242: 1162-1164, 1988), where reaction time could be extended up to 50 hours, several improvements have been done by others (Kim DM, Choi CY: A semicontinous prokaryotic coupled cell-free system using a dialysis membrane, *Biotechnol Prog* 1996, **12**: 645-649; Madin K., Sawasaki T., Ogasawara T., Endo E: A highly efficient and robust cell-free protein synthesis system prepared from wheat embryos: plants apparently contain a suicide system directed at ribosomes, Proc Natl Acad Sci 2000, 97: 559-564).

Summaries of various applications demonstrated that at the moment both types of *in vitro* expression systems, batch mode system [Kawarasaki Y, Nakano H, Yamane T, *Anal Biochem* 1995, **226**: 320-324 ; Nakano H, Tananka T, Kawarasaki Y, Yamane T, *Biosci Biotechnol Biochem* 1994, **58**: 631-634 ; Kim DM, Kigawa T, Chen CY, Yokoyama S, *Eur J Biochem* 1996, **239**: 881-886; Patnaik R, Swartz JR, *BioTechniques* 1998, **24:** 862-868] and systems of continuous actions [Tulin EE, Ken-Ichi T, Shin-Ichiro E, *Biotechnol Bioeng* 1995, **45:** 511-516 ; Kim DM, Choi CY, *Biotechnol Prog* 1996, **12**: 645-649 ; Madin K., Sawasaki T., Ogasawara T., Endo E, Proc Natl Acad Sci 2000, 97: 559-564] are under intensive investigations and developing very fast.

The published and commercially available batch *in vitro* systems are able to synthesized up to 0.5 mg/ml, and the continuous reactors can produce up to 3-5 mg of desired proteins per ml of reaction. In case of wheat germ cell-free continuous expression system the reaction time was extended up to 5 days [Madin K., Sawasaki T., Ogasawara T., Endo E, Proc Natl Acad Sci 2000, 97: 559-564]. In these systems, the continuous removal of the inhibitory byproduct as well as the continuous supply of substrates and energy source enable the continuous reaction system to support protein synthesis over long reaction periods.

The biochemical energy in a cell-free system is supplied by the hydrolysis of triphosphates. For efficient transcription and translation the triphosphate concentration is maintained by an energy regeneration system: creatine phosphate with creatine phosphokinase, phosphoenolpyruvate with pyruvate kinase, and acetyl phosphate with endogenous *E. coli* acetate kinase [Ryabova LA, Vinokurov LM, Shekhovtsova EA, Alakhov YB, Spirin AS, Anal Biochem 1995, 226: 184-186].

Studies of triphosphate levels in different cell-free systems detected a high rate of their hydrolysis, to inorganic phosphate and diphosphates, mainly independently of protein synthesis and these correlates with sudden cessation of protein synthesis [Yao SL, Shen XC, Suzuki E, *J Ferment Bioeng* 1997, 84:7-13 ; Kim RG, Choi CY, J Biotechnol 2000, **84**: 27-32]. The inhibitory effect of accumulating phosphate was also shown directly [Kim D-M, Swartz JR, *Biotechnol Prog* 2000, **16**: 385-390].

Significant modification of ATP-regeneration system for E. *coli in vitro* expression was made by Swartz [Kim D-M, Swartz JR, *Biotechnol and Bioengin* 1999, **66**: 180-188; US patent 6,168,931; US patent 6,337,191] where standard energy sources like acetyl phosphate, phosphoenolpyruvate (PEP) and pyruvate kinase (PK) have been replaced by pyruvate in combination with the enzyme pyruvate oxidase to generate acetyl phosphate. The use of glycolytic intermediates or glucose energy source in combination with NADH and $NAD^+$ was also proposed. Thus, energy source like PEP that also increases the net free phosphate in the reaction mix was omitted. However, the use of glycolytic intermediates, glucose or pyruvate generally support protein synthesis for a period of time no longer than two hours, indicating decrease of NTP's regeneration efficiency or net accumulation of free phosphate.

As shown from the above, the low production protein amount is a main drawback of industrialization of cell-free protein synthesis. Therefore, improvements are still required in terms of total productivity of the protein by increasing the specific production rate and the length of system operation by regenerating an expensive energy source in order to both recycle the regenerated energy source and consume inorganic phosphate which is a strong protein synthesis inhibitor.

### Definitions.

*In vitro* transcription-translation and cell-free transcription-translation refer to any method for cell-free synthesis of a desired protein from DNA encoding the desired protein.

*In vitro* translation and cell-free translation refer to any method for cell-free synthesis of a desired protein from ribonucleic acid (RNA) encoding the desired protein.

Cell-free protein synthesis and *in vitro* protein synthesis refer to both *in vitro* transcription-translation and in vitro translation.

Cell-free extract as used herein denote any preparation comprising the components of a cell's protein synthesis machinery wherein such components are capable of expressing a nucleic acid encoding a desired protein. Thus, a cell free extract comprises components that are capable of translating messenger ribonucleic acid (mRNA)encoding a desired protein.

Biological macromolecules, biological materials and biological polymermolecules refer to i.e. nucleic acids, proteins and fragments thereof.

Cell-free extract enriched with ATP-sulfurylase or cell-free extract containing extra ATP-sulfurylase means that the ATP-sulfurylase concentration is beyond what is usual. The increased or extra amount of ATP-sulfurylase was obtained either by externally adding exogenous ATP-sulfurylase to the cell-free extract or by preparing cell-free extract from cells transformed with a vector over-expressing ATP-sulfurylase.

Cell-free system enriched with ATP-sulfurylase means that the ATP-sulfurylase concentration in the system is beyond what is usual. It comprises either cell-free extract enriched with ATP-sulfurylase and all components used for macromolecules synthesis or standard cell-free extract, all components used for macromolecules synthesis in which extra ATP-sulfurylase was added.

NTPs refer to nucleotide triphosphates (ATP, UTP, GTP and CTP).

### Summary of the invention.

The present invention provides methods for enhancing *in vitro* synthesis of biological macromolecules in a cell-free system where ATP is required as a primary energy source, said method using a cell-free system enriched with ATP-sulfurylase. The invention relates also to cell-free system and cell-free extract enriched with ATP-sulfurylase for enhancing *in vitro* synthesis of any biological macromolecules according to the above method. These biological macromolecules can have various origins (Procaryotic and Eucaryotic).

The enhancing *in vitro* synthesis methods and compositions provided by the invention are useful for *in vitro* production of a wide range of biological polymermolecules such as nucleic acids, proteins and fragments thereof.

ATP-sulfurylase plays several different roles in nature. In fungi, yeasts, most heterotrophic bacteria, algae, and higher plants, ATP-sulfurylase catalyzes the first intracellular reactions in the assimilation of sulfate into reduced organic molecules. In anaerobic sulfate reducing bacteria, ATP sulfurylase forms APS (adenosine 5'-phosphosulfate) solely to serve as the terminal electron acceptor of heterotrophic metabolism. In certain chemo- and photolithotrophic bacteria, ATP sulfurylase catalyzes the last reaction in the oxidation of reduced inorganic sulfur compounds to sulfate. In sulfate-assimilating organism, ATP sulfurylase (ATP: sulfate adenyltransferase, EC 2.7.7.4) catalyzes the first intracellular reaction in the incorporation of inorganic sulfate ($SO^{2-}_4$) into organic molecules, such as adenosine 5'-phosphosulfate (APS) [Karamohamed S et al.: Production, purification, and luminometric analysis of recombinant *saccharomyces cerevisiae* MET3 adenosine triphosphate sulfurylase expressed in *Escherichia coli,* Protein expression and Purification 1999, 15: 381-388].

ATP sulfurylase is widely distributed in nature and has been purified from a number of sources including lower and higher microorganisms, plants and animals. ATP sulfurylase genes have been also cloned from procaryotes, lower eucaryotes, plants, as well as animals.

ATP sulfurylase has been used for many applications such as DNA sequencing (WO9813523A1, Nyren et al. Anal. Biochem., (1985) 151, 504-509). The method is based on the detection of base incorporation by release of pyrophosphate (PPi). In the assay, the PPi which is generated is subsequently converted to ATP by ATP sulfurylase and the ATP production is monitored by luciferase. However, in the assay, ATP production is not used for DNA synthesis but only for the detection process.

ATP-sulfurylase utilizes inorganic phosphate for regeneration of ATP as shown in the scheme hereunder, which is the most commonly used source of energy but the other three NTPs are also used in protein expression and ATP can be used for their regeneration in a cell extract.

$$PPi + APS \quad \xrightleftharpoons{\text{ATP-sulfurylase}} \quad ATP + SO_4^{2-}$$

**Description of the figures.**

Figure 1 shows the synthesis of β-lactamase versus ATP-sulfurylase concentration in an *E. coli* cell-free transcription-translation system from Invitrogen.
Figure 2 shows the synthesis of β-lactamase versus ATP-sulfurylase concentration in an *E. coli* cell-free transcription-translation system from Novagen.

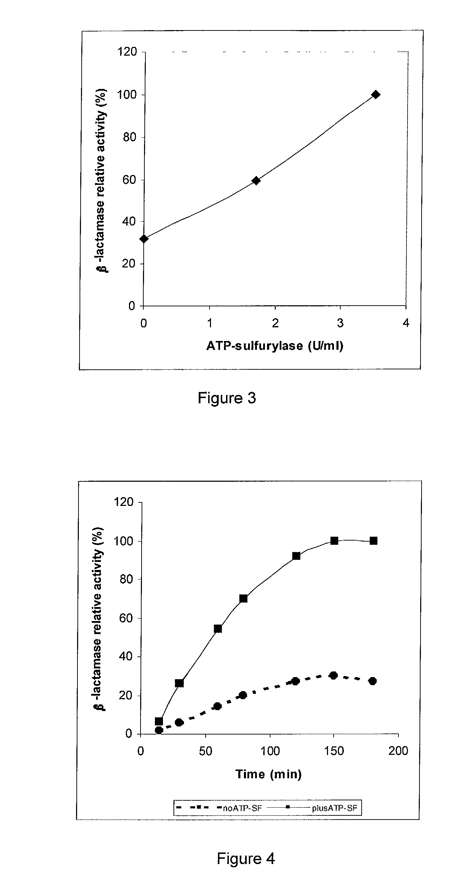

Figure 3 shows the synthesis of β-lactamase versus ATP-sulfurylase concentration in a cell-free protein synthesis system based on the *E. coli* S30 extract constructed according to Kigawa et al (1999)
Figure 4 shows the kinetics of synthesis of β-lactamase in the absence or presence of ATP-sulfurylase in a cell-free translation system based on the *E. coli* S30 extract.

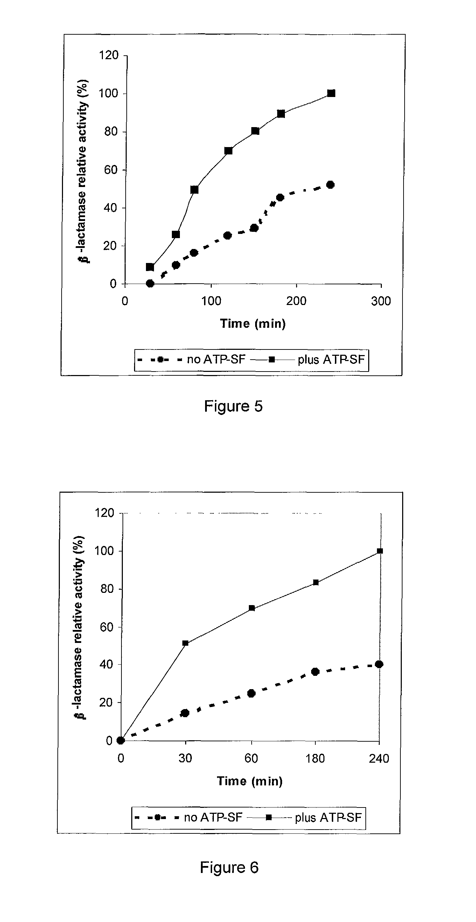

Figure 5 shows kinetics of the synthesis of β-lactamase in the absence or presence of ATP-sulfurylase in a cell-free transcription-translation system based on the *E. coli* S30 extract.
Figure 6 shows the synthesis of β-lactamase in the absence or presence of ATP-sulfurylase in a *Bacillus subtilis* cell-free translation system.

**Detailed description.**

Methods and compositions are provided for enhancing *in vitro* synthesis of biological macromolecules in a cell-free system where ATP is required as a primary energy source. The method comprises synthesizing biological materials in a reaction utilizing NTPs as a primary energy source, wherein NTPs are regenerated using energy regeneration system.

The method according to the present invention is characterized in that the *in vitro* synthesis of biological macromolecules is performed with a cell-free system enriched with ATP-sulfurylase.

ATP-sulfurylase can regenerate ATP by utilization of APS and inorganic phosphate removing inorganic phosphate which causes inhibition of protein synthesis. When ATP-sulfurylase was added to a transcription-translation cell-free system along or with an appropriate concentration of APS, it stimulated protein synthesis for a significant yield. While not limiting to the subject matter of the invention, at least two mechanisms may be proposed for the action of ATP-sulfurylase in a cell-free systems : (1) ATP regeneration and (2) utilization of inorganic phosphate.

Therefore, the cell free reaction system may comprise extra ATP which is regenerated using the cell-free extract enriched with ATP-sulfurylase.

In an other embodiment, the cell free reaction system may comprise exogenous APS.

The *in vitro* synthesis according to the method of the invention relates more particularly to translation of mRNA to produce polypeptides eventually after a transcription of said mRNA from a DNA template. Therefore, the method of the invention further relates to a coupled transcription-translation *in vitro* synthesis. In these embodiments, the cell free system contains all substances necessary for the transcription of mRNA from a DNA template and translation of said mRNA.

The *in vitro* synthesis according to the method of the invention can be carried out in a reaction vessel as a batch reaction.

The *in vitro* synthesis according to the method of the invention can also be carried out continuously or semi-continuously.

Extra ATP-sulfurylase of the cell-free system enriched with ATP-sulfurylase according to the present invention can be derived from prokaryotic organism, eukaryotic organism, transgenic vector, bacterial cell that has been genetically modified, E. *coli* extract, or can be purified. The extract can be prepared from cells transformed with a vector expressing ATP-sulfurylase.

In a first embodiment of the invention, ATP-sulfurylase is added to the cell-free system in order to obtain a cell-free system enriched with ATP-sulfurylase (extra ATP-sulfurylase). More particularly, extra ATP-sulfurylase is added to the cell-free extract.

Extra ATP-sulfurylase can be added to the cell-free system at the beginning and/or during the *in vitro* synthesis. ATP-sulfurylase can be added at intervals during the synthesis.

Extra ATP-sulfurylase can be present in the cell-free system prior to the beginning of the *in vitro* synthesis reaction. For example, extra ATP-sulfurylase can be present in the cell-free extract.

In a second embodiment of the invention, the cell-free system comprises a cell-free extract prepared from cells transformed with a vector over-expressing ATP-sulfurylase.

In practice, ATP-sulfurylase concentration is adapted according to experimental conditions and particularly to the protein to be expressed.

Optimization of ATP-sulfurylase concentration required for an optimal protein expression in a cell-free system can be achieved by titration of ATP-sulfurylase in a range for example from 0.1 to 10 U/ml with or without APS. The concentrations which support maximum of protein production and longer duration time of protein expression in a linear mode can be considered as an optimized for these experimental conditions.

Advantageously ATP-sulfurylase is present in the cell free extract enriched with ATP-sulfurylase at an initial concentration of at least about 0.1 U/ml and more advantageously of about 0.5 U/ml

The present invention relates also to a cell-free system enriched with ATP-sulfurylase for enhancing *in. vitro* synthesis of biological macromolecules according to the above method.

A cell free system enriched with ATP-sulfurylase according to the present invention constitutes a cell free reaction mix for *in vitro* synthesis of biological macromolecules where ATP is required as a primary energy source. It can be prepared from any commercial cell-free reaction mix such as Novagen or Invitrogen, or standard or modified S30 extract by adding extra ATP-sulphurylase to said cell free reaction mix. Therefore, the invention is directed to cell-free system comprising a cell-free extract enriched with ATP-sulfurylase.

The cell-free system can further comprise exogenous APS. It can also comprise all substances necessary for the translation of mRNA and transcription of mRNA from a DNA template.

As indicated above, extra ATP-sulfurylase presents in the cell free extract enriched with ATP-sulfurylase of the invention can be derived from prokaryotic organism, eukaryotic organism, transgenic vector, bacterial cell that has been genetically modified, *E. coli* extract, or can be purified.

The cell-free extract enriched with ATP-sulfurylase can be prepared from cells transformed with a vector over-expressing ATP-sulfurylase.

Advantageously, ATP-sulfurylase is present in a concentration of at least about 0.1 U/ml and more preferentially at least about 0.5 U/ml.

An example of a cell-free system on the basis of which can be prepared a cell-free system enriched with ATP-sulfurylase according to the present invention comprises :

- cell-free extract which contains the enzymes and factors necessary for transcription and translation;
- monomers for the macromolecule to be synthesized (e.g. amino acids, nucleotides, etc...),
- an energy regeneration system like acetyl phosphate (AcPh) and acetate kinase phosphoenolpyruvate (PEP) and pyruvate kinase or creatine phosphate (CrPh) and creatine kinase etc,and
- eventually a template for production of macromolecule, e.g. DNA and/or mRNA etc.

Such cell-free systems are well-known in the art, and have been described in the literature (see for example Kigawa et al., 1999).

In the invention the cell-free system is further enriched with ATP-sulfurylase.

The invention also relates to a cell-free extract enriched with ATP-sulfurylase according to the above cell-free system enriched with ATP-sulfurylase.

An example of a cell-free extract on the basis of which can be prepared a cell-free extract enriched with ATP-sulfurylase according to the present invention comprises the enzymes, factors and components of a cell's protein synthesis machinery e.g. ribosomes, tRNA, polymerases, transcriptional factors, etc.

Such cell-free extracts are well-known in the art, and have been described in the literature (Zubay G et al. (1973) *In vitro* synthesis of proteins in microbial system, *Annu Rev Genet,* **7**:267287 ; Roberts BE et al. (1973) Efficient translation of tobacco mosaic virus RNA and rabbit globin 9S RNA in a cell-free system from commercial wheat germ, *Proc Natl Acad Sci USA,* **70**: 2330-2334 ; Pelham HRB et al. (1976) An efficient mRNA-dependent translation system from reticulocyte lysates, *Eur J Biochem,* **67**:247-256 ; Shimizu Y. et al (2001) Cell-free translation reconstituted with purified components, Biotech. Nature, **19**, 751-755).

## Examples.

The following examples are offered to illustrate but not to limit the present invention.

Example 1 : Synthesis of [β-lactamase versus ATP-sulfurylase concentration in a cell-free transcription-translation system from Invitrogen.

The standard reaction mixture of 50 µl for cell-free transcription-translation system employing the ATP-sulfurylase-dependent utilization of inorganic phosphate and ATP regeneration consist of the following components in 50 µl : 25 µl of IVPS *E. coli* Extract (composition proprietary of Invitrogen, Expressway in vitro protein synthesis system E. coli. Cat n°. 12333.019), 20 µl of 2.5xIVPS *E. coli* reaction buffer (composition proprietary of Invitrogen), 1 µl of T7 RNA polymerase (composition proprietary of Invitrogen), and 0.1 mg/ml of PCR product encoding for β-lactamase.

In this reaction, ATP-sulfurylase was added in concentrations of 1.7 and 3.5 U/ml. Reactions were conducted for 2 h in a water bath set at 37°C according to manufacturer's instructions (Invitrogen). Detection of β-lactamase activity was done by nitrocefin test as described by O'Callaghan et al. [O'Callaghan C.H., Morris S.M., Kirby S.M., Shingler A.H, *Antimicrob. Agents Chemother* 1972, 1: 283-288]. 50 µl from cell-free transcription-translation reaction mix, diluted 600 fold, was mixed with 150 µl of nitrocefin (16 µg/ml) in a well of a microtitration plate and OD at 486 nm was measured. S. cerevisiae ATP-sulfurylase was from Sigma.

The use of exogenous ATP-sulfurylase seems to be significant in the expression of proteins particularly sensitive to the concentration of inorganic phosphate. The final yield of β-lactamase was 3 fold that of the experiment carried out without added extra ATP-sulfurylase.

Example 2 : Synthesis of β-lactamase versus ATP-sulfurylase concentration in an *E. coli* cell-free transcription-translation system from Novagen.

The standard reaction mixture of 50 µl for cell-free transcription-translation system employing the ATP-sulfurylase-dependent utilization of inorganic phosphate and ATP regeneration consist of the following components in 50 µl: 25 µl of IVPS E. *coli* Extract (composition proprietary of Novagen, Introductory EcoPro<sup>TM</sup> T7 System. Cat. N°. 70888-3), 20 µl of 2.5xIVPS *E. coli* reaction buffer (composition proprietary of Novagen), and 0.1 mg/ml of PCR product or 0.2 mg/ml of plasmid DNA, both encoding for β-lactamase.

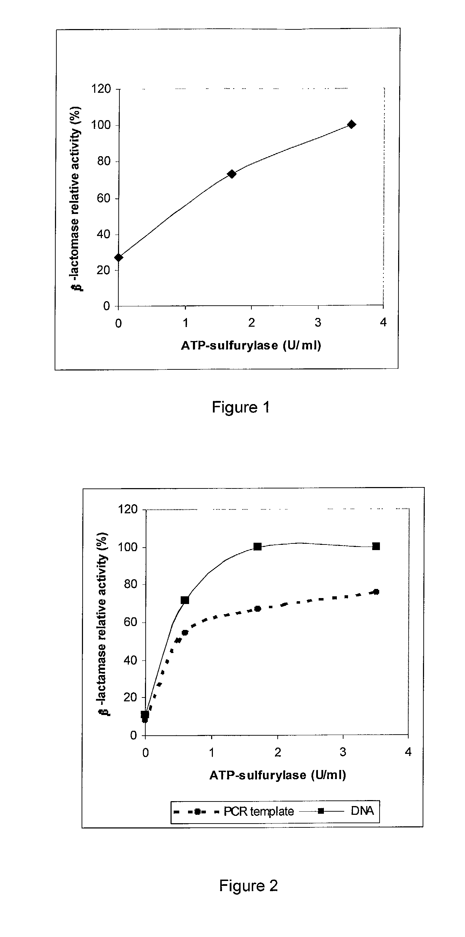

In this commercial extract ATP-sulfurylase was added in concentrations of 0.6, 1.7 and 3.5 U/ml. Reactions were conducted for 1 h in a water bath set at 37°C according manufacture instructions (Novagen). Detection of (β-lactamase activity was done by nitrocefin test (see Fig. 1). 50 µl from the cell-free transcription-translation reaction mix, diluted 40 fold, was mixed with 150 µl of nitrocefin (16 µg/ml) in a well of a microtitration plate and OD at 486 nm was measured. S. cerevisiae ATP-sulfurylase was from Sigma.

The effect of added ATP-sulfurylase on the β-lactamase expression level is striking, reaching a 10-fold higher level than that without ATP-sulfurylase in the *E. coli* cell-free transcription-translation system from Novagen.

Example 3 : Synthesis of β-lactamase versus ATP-sulfurylase concentration in a cell-free protein synthesis system constructed according Kigawa et al. (1999).

The standard reaction mixture of 50 µl for cell-free transcription-translation system as described by Kigawa (1999) with 0.3 volumes of S30 extract was used. In the S30 extract ATP-sulfurylase was added in concentrations of 1.7 and 3.5 U/ml. Reactions were conducted for 3 h in a water bath set at 30°C. 50 µl from cell-free transcription-translation reaction mix, diluted 600 fold, was mixed with 150 µl of nitrocefin (16 µg/ml) in a well of a microtitration plate and OD at 486 nm was measured.

S30 E. *coli* extract was prepared as described by (Zubay G. (1973) 7 RNA polymerase was from (Hybaid), S. cerevisiae ATP-sulfurylase was from Sigma.

The ATP-sulfurylase addition into the E. *coli* cell-free transcription-translation system prepared as described by Kigawa et al (1999) increases the level of β-lactamase expression up to 3-fold, similar to the result with the Invitrogen mix.

Table 1 hereunder reports the stimulation of β-Lactamase expression by addition of ATP-Sulfurylase (ATP-SF) in different transcription-translation cell-free systems programmed with PCR templates. βLactamase expression is expressed in relative activity (%).

Table 1

| | Cell-free systems / ATP-SF, U/ml | | | |
|---|---|---|---|---|
| | no | 0.6 | 1.7 | 3.5 |
| Invitrogen | 27% | | 73% | 100% |
| Novagen | 10.6% | 71.3% | 88.4% | 100% |

Table 1   (continued)

| | Cell-free systems / ATP-SF, U/ml | | | |
|---|---|---|---|---|
| | no | 0.6 | 1.7 | 3.5 |
| Kigawa et al | 32% | | 59% | 100% |

Example 4 : Kinetics of synthesis of β-lactamase in the absence or presence of ATP-sulfurylase in an E. *coli* cell-free translation system.

The standard reaction mixture of 50 µl for cell-free translation system as described by Kigawa (1999) -with 0.3 volumes of S30 extract was used.

In the S30 extract prepared as described by Zubay et al. (1973) ATP-sulfurylase was added in concentrations of 1.5 U/ml. Reactions were conducted in a water bath set at 30°C. 50 µl taken from cell-free transcription-translation reaction mix at different time points, diluted 600 fold, were mixed with 150 µl of nitrocefin (16 µg/ml) in a well of a microtitration plate and OD at 486 nm was measured.

*S. cerevisiae* ATP-sulfurylase was from Sigma.

Table 2 hereunder reports the β-lactamase relative activity (%)versus reaction time in the absence and presence of ATP-sulfurylase.

Table 2

| | β-lactamase relative activity (%) in *E. coli* cell-free translation system | |
|---|---|---|
| Reaction time (min) | Without ATP-SF | With ATP-SF |
| 15 | 1.8 | 6.3 |
| 30 | 5.4 | 26 |
| 60 | 14 | 54 |
| 80 | 20 | 70 |
| 120 | 27 | 92 |
| 150 | 30 | 100 |
| 180 | 27 | 100 |

The kinetics of β-lactamase cell-free synthesis show that a 3-fold increase in protein production could be observed after 30 minutes of translation system incubation.

Example 5 : Synthesis of [β-lactamase in the absence and presence of ATP-sulfurylase from *S. cerevisiae* in an *E. coli* cell-free transcription-translation system.

The standard reaction mixture of 50 µl for cell-free translation system as described by Kigawa (1999) employing the ATP-sulfurylase-dependent utilization of inorganic phosphate and ATP regeneration consist of the following components in 50 µl: 60 mM Hepes pH 8.2, 1.2 mM ATP, 0.8 mM GTP, 1 mM DTT, 0.64 mM cAMP, 200 mM potassium glutamate, 80 mM NH4 (OAc) , 6 mM Mg (OAc) 2, 34 µg/µl folinic acid, 0.2 mg/ml of PCR template, 1 U/µl T7 polymerase with 0.3 volumes of S30 was used. In the reaction mixture ATP-sulfurylase was added in concentrations of 1.5 U/ml. Reactions were conducted for 3 h in a water bath set at 30°C. 50 µl aliquotes were taken from cell-free transcription-translation reaction mix at different time points, diluted 600 fold, were mixed with 150 µl of nitrocefin (16 µg/ml) in a well of a microtitration plate and OD at 486 nm was measured.

T7 RNA polymerase was from (Hybaid), S. *cerevisiae* ATP-sulfurylase was from Sigma.

Table 3 hereunder reports the β-lactamase relative activity (%) versus reaction time in the absence and presence of ATP-sulfurylase.

Table 3

| | β-lactamase relative activity (%) in *E. coli* cell-free transcription-translation system | |
|---|---|---|
| Reaction time (min) | Without ATP-SF | With ATP-SF |
| 30 | 4.7 | 8.6 |
| 60 | 10 | 26 |
| 80 | 16.2 | 49 |
| 120 | 25 | 70 |
| 150 | 29 | 80 |
| 180 | 45 | 89 |
| 240 | 52 | 100 |

These experiments show that ATP-sulfurylase has a great effect on β-lactamase synthesis. Indeed, β-lactamase production after one hour of reaction was 3 fold greater than the one obtained without ATP-sulfurylase.

Example 6 : Synthesis of β-lactamase with and without ATP-sulfurylase in a *Bacillus subtilis* cell-free translation system.

The standard reaction mixture of 50 µl for cell-free translation system employing the ATP-sulfurylase-dependent utilization of inorganic phosphate and ATP regeneration as described by Kigawa (1999) with 0.3 volumes of S30 extract prepared as described by Chambliss et al. (Chambliss G. H. et al. : Bacteria *in vitro* protein-synthesizing systems; Methods in enzymology (1983), **101**, 598-605). In the S30 extract ATP-sulfurylase was added in concentrations of 1 U/ml. Reactions were conducted for 3 h in a water bath set at 30°C. 50 µl aliquots were taken from cell-free transcription-translation reaction mix at different time points, diluted 500 fold, were mixed with 150 µl of nitrocefin (16 µg/ml) in a well of a microtitration plate and OD at 486 nm was measured.

*S. cerevisiae* ATP-sulfurylase was from Sigma.

Table 4 hereunder reports the β-lactamase relative activity (%) in the cell-free translation system based on Bacillus subtilis extract in the absence and presence of ATP sulfurylase

Table 4

| Time (min) | Cell-free translation without ATP-SF | Cell-free translation with ATP-SF |
|---|---|---|
| 30 | 14,4 | 51 |
| 60 | 24,5 | 69,4 |
| 180 | 36,1 | 83 |
| 240 | 40,4 | 100 |

The production of β-lactamase in the *Bacillus subtilis* transcription-translation system in the presence of ATP-sulfurylase was increased up to 2.5 fold compared to the one obtained without ATP-sulfurylase. The effect was observed after 30 minutes of incubation.

**Claims**

1. A method for enhancing *in vitro* synthesis of biological macromolecules in a cell-free system where ATP is required as a primary energy source, wherein said cell-free system is enriched with ATP-sulfurylase

2. The method of claim 1, wherein the biological macromolecules are polymermolecules such as nucleic acids, proteins and fragments thereof.

3. A method according to anyone of claims 1 or 2, wherein the cell free system comprises extra ATP.

4. A method according to anyone of claims 1 or 2, wherein the cell free system comprises exogenous APS.

**5.** A method according to anyone of claims 1 to 4, wherein said *in vitro* synthesis comprises translation of mRNA to produce polypeptides.

**6.** The method of claim 5, wherein said *in vitro* synthesis comprises also transcription of mRNA from a DNA template.

**7.** A method according to anyone of claims 1 to 6, wherein said *in vitro* synthesis is carried out in a reaction vessel as a batch reaction.

**8.** A method according to anyone of claims 1 to 8, wherein said *in vitro* synthesis is carried out semi continuously or continuously.

**9.** A method according to anyone of claims 1 to 8, wherein ATP-sulfurylase is added to the cell-free system at the beginning and/or during the *in vitro* synthesis.

**10.** The method of claim 9, wherein extra ATP-sulfurylase is added to the cell-free system at intervals during the *in vitro* synthesis.

**11.** A method according to anyone of claims 1 to 8, wherein the cell-free system comprises a cell-free extract prepared from cells transformed with a vector over-expressing ATP-sulfurylase.

**12.** A method according to anyone of claims 1 to 11, wherein ATP-sulfurylase concentration is adapted according to the experimental conditions and the biological macromolecules to be synthesized.

**13.** A method according to anyone of claims 1 to 12, wherein ATP-sulfurylase is present in the cell-free system at an initial concentration of at least about 0.1 U/ml.

**14.** The method of claim 13, wherein ATP-sulfurylase is present in the cell-free system at an initial concentration of at least about 0.5 U/ml.

**15.** A cell-free system enriched with ATP-sulfurylase.

**16.** A cell-free system according to claim 15 comprising exogenous APS.

**17.** A cell-free system according to anyone of claims 15 or 16 comprising all substances necessary for the translation of mRNA and transcription of mRNA from a DNA template.

**18.** A cell-free system according to anyone of claims 15 to 17 comprising a cell-free extract enriched with ATP-sulfurylase.

**19.** A cell-free system according to anyone of claims 15 to 17, wherein extra ATP-sulfurylase is derived from prokaryotic organism, eukaryotic organism, transgenic vector, bacterial cell that has been genetically modified, *E. coli* extract, or is purified.

**20.** A cell-free system according to claim 18, wherein the cell-free extract enriched with ATP-sulfurylase is prepared from cells transformed with a vector over-expressing ATP-sulfurylase.

**21.** A cell-free system according to anyone of claims 15 to 20, wherein ATP-sulfurylase is present in a concentration of at least about 0.1 U/ml.

**22.** A cell-free system according to claim 21, wherein ATP-sulfurylase is present in a concentration of at least about 0.5 U/ml.

**23.** A cell-free extract enriched with ATP-sulfurylase.

**24.** A cell-free extract according to claim 23 comprising exogenous APS.

**25.** A cell-free extract according to anyone of claims 23 or 24 comprising all substances necessary for the translation of mRNA and transcription of mRNA from a DNA template.

26. A cell-free system according to anyone of claims 23 to 25, wherein extra ATP-sulfurylase is derived from prokaryotic organism, eukaryotic organism, transgenic vector, bacterial cell that has been genetically modified, *E. coli* extract, or is purified.

27. A cell-free system according to anyone of claims 23 to 25 prepared from cells transformed with a vector over-expressing ATP-sulfurylase.

28. A cell-free extract according to anyone of claims 23 to 27, wherein ATP-sulfurylase is present in a concentration of at least about 0.1 U/ml.

29. A cell-free system according to claim 28, wherein ATP-sulfurylase is present in a concentration of at least about 0.5 U/ml.

Figure 1

Figure 2

Figure 3

Figure 4

Figure 5

Figure 6

**EP 1 386 962 A1**

European Patent
Office

**EUROPEAN SEARCH REPORT**

Application Number

EP 02 29 1959

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 22615 A (LIFE TECHNOLOGIES INC) 28 May 1998 (1998-05-28) <br><br> * example 2 * <br> --- | 1-4, 7-16, 18-24, 26-29 | C12N9/12 <br> C12P19/34 <br> C12P21/02 |
| A | KIM DONG-MYUNG ET AL: "Regeneration of adenosine triphosphate from glycolytic intermediates for cell-free protein synthesis." <br> BIOTECHNOLOGY AND BIOENGINEERING, vol. 74, no. 4, 20 August 2001 (2001-08-20), pages 309-316, XP002227479 <br> ISSN: 0006-3592 <br> * the whole document * <br> ----- | 1-29 | |
| | | | TECHNICAL FIELDS SEARCHED (Int.Cl.7) <br><br> C12N <br> C12P |

The present search report has been drawn up for all claims

| Place of search | Date of completion of the search | Examiner |
|---|---|---|
| THE HAGUE | 24 February 2003 | Lejeune, R |

EPO FORM 1503 03 82 (P04C01)

**ANNEX TO THE EUROPEAN SEARCH REPORT
ON EUROPEAN PATENT APPLICATION NO.**     EP 02 29 1959

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2003

| Patent document cited in search report | Publication date | Patent family member(s) | Publication date |
|---|---|---|---|
| WO 9822615 A | 28-05-1998 | US 6291164 B1<br>AU 7302198 A<br>WO 9822615 A1<br>US 2001055792 A1 | 18-09-2001<br>10-06-1998<br>28-05-1998<br>27-12-2001 |

EPO FORM P0459